# EUROPÄISCHE PATENTSCHRIFT

(11) **EP 1 272 116 B1**
(45) Veröffentlichungstag und Bekanntmachung des Hinweises auf die Patenterteilung: **29.09.2004**
(21) Anmeldenummer: 00914005.4
(22) Anmeldetag: 10.04.2000
(51) Int. Cl.: A61B 17/74, A61B 17/72

(54) **OSTEOSYNTHETISCHES VERANKERUNGSELEMENT**
OSTEOSYNTHETIC ANCHORING ELEMENT
ELEMENT D'ANCRAGE OSTEOSYNTHETIQUE

(43) Veröffentlichungstag der Anmeldung: 08.01.2003
(73) Patentinhaber: SYNTHES AG Chur, 7002 Chur (CH)
(72) Erfinder: FREI, Reto, CH-7270 Davos (CH); HEHLI, Markus, CH-7276 Frauenkirch (CH)
(74) Vertreter: Lusuardi, Werther Giovanni, Dr.
(86) Internationale Anmeldenummer: PCT/CH2000/000207
(87) Internationale Veröffentlichungsnummer: WO 2001/076493

(56) Entgegenhaltungen:
- DE-A- 19 612 276
- US-A- 3 716 051
- US-A- 3 805 775
- US-A- 4 236 512
- US-A- 4 379 451

## Beschreibung

Die Erfindung bezieht sich auf ein osteosynthetisches Verankerungselement gemäss dem Oberbegriff des Patentanspruchs 1 und auf eine Spannvorrichtung mit einem solchen Verankerungselement 3 zur Fixation von abgebrochenen Hüftgelenkköpfen gemäss dem Patentanspruch 23.

Bei der osteosynthetischen Behandlung von Schenkelhalsbrüchen, d.h. solchen Brüchen des Femur, bei denen der den Gelenkkopf mit dem restlichen Teil des Femur verbindende Schenkelhals abgebrochen ist, werden die Knochenfragmente temporär durch eine Spannvorrichtung miteinander verbunden. Eine solche Spannvorrichtung mit
a) einer in den abgebrochenen Gelenkkopf einzuschraubenden Ankerschraube, welche über einen kopflosen Schaft mit einem Innengewinde am hinteren Ende verfügt;
b) einer am Hauptteil des Femur festzuschraubenden Platte mit einer Buchse zur Aufnahme des Schaftes der Ankerschraube; und
c) einer in das Innengewinde am hinteren Ende der Verankerungsschraube einschraubbaren Kompressionsschraube, deren Schraubenkopf auf der hinteren Schulterfläche der Buchse aufliegt,
ist beispielsweise aus der CH 634 742 SUTTER bekannt. Nachteilig bei dieser Art von Ankerschrauben ist, dass diese beim Anziehen der Kompressionsschraube nicht mitdrehen dürfen, da sonst die Ankerschraube weiter in den Gelenkkopf eingedreht würde und nicht wie gewünscht, die Knochenfragmente aufeinander zu bewegt. Aus diesem Grund müssen die Ankerschrauben in der Buchse mittels prismatischer Form des Schaftes der Ankerschrauben und der Bohrungen in der Buchse oder mittels Stift/Nuten-Verbindungen zwischen Ankerschraube und Buchse am Mitdrehen gehindert werden. Dadurch entsteht für den Chirurgen während der Implantation der Spannvorrichtung das Problem, die Platte mit der Buchse nach dem Eindrehen der Ankerschraube passgenau zur Verdrehsicherung über diese zu schieben. Besonders erhöht wird diese Schwierigkeit, weil die Ankerschraube nicht über den Knochen vorsteht sondern ca. 10 mm in diesem vertieft ist, was zu einer Blindmanipulation und zeitraubendem Suchen nach der passenden Stellung der Buchse führt.

In der DE 196 12 276 TRAUTMANN wird eine Vorrichtung zur operativen Versorgung von Brüchen offenbart, welche sich dadurch auszeichnet, dass sie einen Schaft mit mehreren elastisch spreizbaren Spreizdrähten umfasst, wobei die Spreizdrähte mittels einer Betätigungseinrichtung nach dem Einführen des Schaftes in einen vorgängig erstellten Bohrkanal quer zur Längsachse des Schaftes ausbiegbar sind. Die Spreizdrähte werden in Sacklochausnehmungen eingebracht, welche die vorderen Enden der Spreizdrähte umschliessen. Damit biegen sich die Spreizdrähte beim Betätigen der Spannmittel mit einer Biegelinie aus, welche gegen das vordere Ende der Spreizdrähte S-förmig verläuft. Durch diese S-förmige Ausbiegung an den vorderen Enden der Spreizdrähte wird die Verankerung des Verankerungselementes vor allem in der Nähe der Spitze verringert, was speziell bei der Anwendung des Verankerungselementes bei einer Fixierung eines Hüftgelenkkopfes wegen der kurzen Verankerungslänge ungünstig ist.

Hier will die Erfindung Abhilfe schaffen. Der Erfindung liegt die Aufgabe zugrunde, ein osteosynthetisches Verankerungselement zu schaffen, welches ein einfaches Überschieben der Buchse über das bereits gesetzte Verankerungselement gestattet, eine Verdrehsicherung zwischen Buchse und Verankerungselement zur Verhinderung einer Rotation des Hüftgelenkkopfes umfasst und zudem die physiologisch auftretenden Belastungen optimal aufnimmt. Die Optimierung der Belastungsaufnahme kann dank der Erfindung ohne Dimensionsvergrösserung des Verankerungselementes erfolgen.

Die Erfindung löst die gestellte Aufgabe mit einem osteosynthetischen Verankerungselement, welches die Merkmale des Anspruchs 1 aufweist, sowie mit einer Spannvorrichtung mit einem solchen Verankerungselement, welche die Merkmale des Anspruchs 23 aufweist.

Weitere vorteilhafte Ausgestaltungen der Erfindung sind in den abhängigen Ansprüchen gekennzeichnet.

Das erfindungsgemässe osteosynthetische Verankerungselement umfasst einen prismatischen oder zylindrischen Schaft vom Durchmesser D, im Schaft gelagerte, elastisch und bezüglich des Schaftes radial ausknickbare Verankerungsmittel sowie Spannmittel, welche am hinteren Ende des Schaftes angebracht sind und mittels welcher die Verankerungsmittel reversibel ausknickbar sind und damit das Verankerungselement im Knochen verankerbar ist. Das Verankerungselement weist eine Längsachse, ein vorderes in einen Knochen einbringbares Ende und ein hinteres in eine interne Platte oder ein Verbindungselement einbringbares Ende auf.

Die Verankerungsmittel lassen sich mittels der Spannmittel bogenförmig bis zu einem Durchmesser Dₘₐₓ > D ausbiegen. Das Verhältnis Dₘₐₓ : D kann zwischen 1,2 und 3, vorzugsweise zwischen 1,5 und 2,5 betragen. Das Ausknicken der Verankerungsmittel erfolgt vorteilhafterweise auf einer Länge L, welche zwischen 10 und 60 mm beträgt. Bevorzugt sind die Verankerungsmittel elastisch ausbiegbar. Für spezielle Anwendungen sind jedoch auch plastisch deformierbare Verankerungsmittel denkbar.

In einer bevorzugten Ausführungsform des erfindungsgemässen osteosynthetischen Verankerungselementes sind die Verankerungsmittel als Verankerungsdrähte mit einem Durchmesser d und je einem hinteren, spannmittelseitigen Ende und einem, longitudinal zum Schaft betrachtet, entgegengesetztem vorderen Ende ausgebildet. Vorzugsweise sind 3 bis 6 Verankerungsdrähte an einem Verankerungselement angebracht.

Die Verankerungsdrähte sind parallel zur Längsachse angeordnet, wobei beide Enden im Schaft gelagert sind und bei Betätigung des Spannmittels die Verankerungsdrähte zwischen ihren Enden senkrecht zur Längsachse bogenförmig ausbiegbar sind.

Der Durchmesser d der Verankerungsdrähte beträgt zwischen 0,5 mm und 2,5 mm, vorzugsweise zwischen 1 mm und 1,5 mm beträgt. Die Enden der Verankerungsdrähte sind vorteilhafterweise kugelig ausgestaltet, wobei vorzugsweise der Kugeldurchmesser grösser als der Durchmesser d ist.

In einer weiteren Ausführungsform des erfindungsgemässen osteosynthetischen Verankerungselementes ist das Spannmittel als Spindel ausgeführt, welche koaxial zur Längsachse im Schaft angeordnet ist und deren Aussengewinde in ein entsprechendes Innengewinde einer Gewindehülse schraubbar ist, welche im hinteren Schaftabschnitt in einer konzentrisch zur Längsachse im Schaft verlaufenden Bohrung axialfest und rotationsfest gelagert ist. Die Spindel umfasst gegen den vorderen Schaftabschnitt hin ein in der Bohrung parallel zur Längsachse verschiebbares Lagerteil mit einer Ringnute. Die Ringnute ist am Lagerteil in einem Querschnitt senkrecht zur Längsachse angeordnet und dient zur Aufnahme der hinteren Enden der Verankerungsdrähte. Das Lagerteil ist axialfest, aber um die Längsachse rotierbar mit der Spindel verbunden. An der Mantelfläche der Bohrung im Schaft ist eine parallel zur Längsachse verlaufende Nute angeordnet, in welche ein am Lagerteil radial vorstehender Stift eingreift. Durch die Anordnung der Nute in der Bohrung des Schaftes und des Stiftes am Lagerteil wird erreicht, dass das Lagerteil durch die Spindel axial verschiebbar ist, gegenüber dem Schaft aber gegen Rotation gesichert ist. Dadurch wird verhindert, dass auf die Verankerungsdrähte beim Drehen der Spindel ein Drehmoment ausgeübt wird. Zum Drehen der Spindel können am hinteren Ende derselben Mittel zur Aufnahme eines Schraubendrehers, beispielsweise ein Innensechskant oder auch ein Schlitz angebracht sein. Ferner kann die Spindel am hinteren Ende mit einer koaxialen Bohrung mit Innengewinde zur Aufnahme einer Kompressionsschraube ausgestattet sein.

Am vorderen Ende des Schaftes wird vorteilhafterweise ein Zapfen teilweise in die Bohrung eingefügt, welcher vom vorderen Schaftende weg gerichtet konvex, vorzugsweise sphärisch ausgebildet sein kann, wobei der konvexe Teil des Zapfens den vorderen Abschluss des Verankerungselementes bildet. An seinem in die Bohrung ragenden Teil kann ebenfalls eine Ringnute, welche in einer senkrecht zur Längsachse stehenden Ebene verläuft, zur Aufnahme der vorderen Enden der Verankerungsdrähte angebracht sein. Beide Ringnuten weisen vorteilhafterweise einen kreisförmigen Querschnitt auf.

Am hinteren Schaftende können wiederum Mittel zur Aufnahme eines Schraubendrehers, beispielsweise in Form eines Schlitzes angebracht sein. Damit kann beim Drehen der Spindel der Schaft durch Entgegenhalten mit einem Schraubenzieher gegen Mitrotieren mit der Spindel gesichert werden.

Die erfindungsgemässe Spannvorrichtung dient zum Fixieren abgebrochener Hüftgelenkköpfe und umfasst neben einem erfindungsgemässen Verankerungselement, welches in der Spongiosa des abgebrochenen Gelenkkopfes durch Ausbiegen der Verankerungsdrähte befestigt wird, eine am Hauptteil des Femur festzuschraubende Platte mit einer Buchse, worin das Verankerungselement auf einem an sein hinteres Ende anstossenden Teil seiner Länge aufnehmbar und koaxial zur Längsachse verschiebbar ist sowie eine in das Innengewinde der Spindel am hinteren Ende des Schaftes einschraubbare Kompressionsschraube, deren Kopf auf einer Schulterfläche an der Buchse aufliegt. Mit Hilfe dieser Kompressionsschraube lässt sich der abgebrochene Femurkopf an den Schenkelhals heranziehen. Zwischen dem Verankerungselement und der Buchse ist eine Verdrehsicherung angebracht, wodurch eine Rotation des Hüftgelenkkopfes um die Längsachse des Verankerungselementes verhindert wird.

Die durch die Erfindung erreichten Vorteile sind im wesentlichen darin zu sehen, dass dank des erfindungsgemässen osteosynthetischen Verankerungselementes dieses durch die elastisch und radial ausknickbaren Verankerungsmittel innerhalb eines grossen Volumens im Knochen verankert wird. Dies ist vor allem bei Knochen, welche von Osteoporose befallen sind, vorteilhaft. Zudem lässt sich die Platte mit der Buchse einfach über das bereits im Knochen verankerte, erfindungsgemässe Verankerungselement schieben, was eine wesentliche einfachere Implantation der gesamten Spannvorrichtung gestattet.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 eine Ansicht der bevorzugten Ausführungsform des erfindungsgemässen osteosynthetischen Verankerungselementes;
Fig. 2 eine Ansicht von der Plattenseite auf die in Fig. 1 dargestellte Ausführungsform des erfindungsgemässen osteosynthetischen Verankerungselementes;
Fig. 3 einen Längsschnitt durch ein im Femur implantiertes erfindungsgemässes Verankerungselement als Bestandteil einer Ausführungsform der Spannvorrichtung zum Fixieren eines abgebrochenen Hüftgelenkkopfes;
Fig. 4 einen Längsschnitt durch ein im Femur implantiertes erfindungsgemässes Verankerungselement als Bestandteil einer anderen Ausführungsform der Spannvorrichtung zum Fixieren eines abgebrochenen Hüftgelenkkopfes; und
Fig. 5 einen Schnitt durch die in Fig. 4 dargestellte Ausführungsform der Spannvorrichtung.

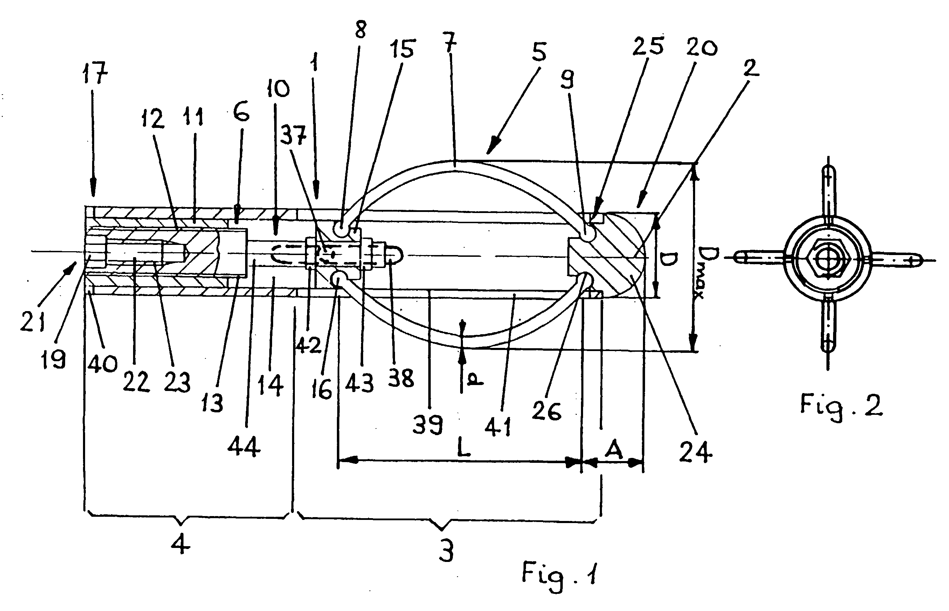

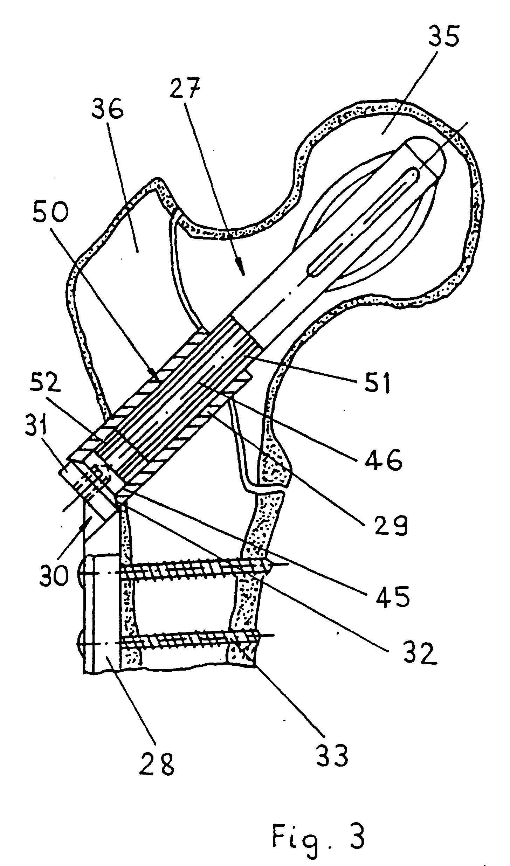

Fig. 1 und 2 zeigen eine Ausführungsform des erfindungsgemässen osteosynthetischen Verankerungselementes mit einem zylindrischen Schaft 1 vom Durchmesser D und mit der Längsachse 2, einem vorderen, in den Knochen einbringbaren Schaftabschnitt 3 und einen in eine interne Platte 28 oder ein anderes Verbindungselement einbringbaren hinteren Schaftabschnitt 4. Der Schaft 1 ist konzentrisch mit einer Bohrung 14 durchbohrt und die verbleibende Rohrwand ist am vorderen Schaftabschnitt 3 mit parallel zur Längsachse 2 verlaufenden Schlitzen 41 versehen, welche die Rohrwand radial durchdringen. Durch diese Schlitze 41 sind radial und elastisch bogenförmig ausbiegbare Verankerungsdrähte 7 führbar. Die Verankerungsdrähte 7 sind mittels eines im Schaft 1 angeordneten Spannmittels 6 durch diese Schlitze 41 ausbiegbar oder umgekehrt auch in ihre Ausgangslage rückführbar. Diese Verankerungsdrähte 7 weisen einen Durchmesser d, parallel zur Längsachse 2 betrachtet ein vorderes Ende 9 und ein hinteres Ende 8 auf. Diese Enden 8;9 sind kugelig ausgestaltet, wobei die Kugeldurchmesser grösser als der Durchmesser d sind. Am vorderen Ende 20 des Schaftes 1 ist ein Zapfen 24 teilweise in die Bohrung 14 eingefügt, welcher koaxial vom Schaft 1 weg sphärisch ausgebildet ist und den vorderen Abschluss des Verankerungselementes bildet. Am in die Bohrung 14 ragenden zylindrischen Abschnitt 25 des Zapfens 24 ist eine Ringnute 26 angebracht, welche in einer senkrecht zur Längsachse 2 stehenden Ebene verläuft. Diese Ringnute 26 weist einen kreisförmigen Querschnitt auf und dient zur Aufnahme der vorderen Enden 9 der Verankerungsdrähte 7. Als Spannmittel 6 dient eine Spindel 10, welche koaxial zur Längsachse 2 im Schaft 1 angeordnet ist und deren Aussengewinde 13 in ein entsprechendes Innengewinde 12 einer Gewindehülse 11 schraubbar ist. Die Gewindehülse 11 ist im hinteren Schaftabschnitt 4 in der konzentrisch zur Längsachse 2 im Schaft 1 verlaufenden Bohrung 14 axialfest und rotationsfest angeordnet. Die Spindel 10 umfasst gegen den vorderen Schaftabschnitt 3 hin ein in der Bohrung 14 parallel zur Längsachse 2 verschiebbares Lagerteil 15 mit einer Ringnute 16, welche auf dem Lagerteil 15 in einer senkrecht zur Längsachse 2 stehenden Ebene angeordnet ist und zur Aufnahme der hinteren Enden 8 der Verankerungsdrähte 7 dient. Das Lagerteil 15 ist koaxial durchbohrt und auf einem gegen den vorderen Schaftabschnitt 3 gerichtet an das Aussengewinde 13 der Spindel 10 koaxial anschliessenden zylindrischen Abschnitt 44 der Spindel 10 drehbar gelagert. Gegen das Aussengewinde 13 der Spindel 10 gerichtet ist dieses Lagerteil 15 mittels eines Wulstes 42, und gegen den vorderen Schaftabschnitt 3 hin beispielsweise mittels eines Seegeringes 43 axialfest, aber um die Längsachse 2 rotierbar auf der Spindel 10 befestigt. Anstelle des Seegerringes 43 könnte auch eine Mutter, welche auf ein entsprechendes Aussengewinde am zylindrischen Abschnitt 44 der Spindel 10 schraubbar ist, zur Befestigung des Lagerteils 15 auf der Spindel 10 dienen. Damit beim Anziehen dieser Mutter das Lagerteil 15 nicht zwischen Mutter und Wulst 42 festgeklemmt würde, kann am zylindrischen Abschnitt 44 zwischen Wulst 42 und Aussengewinde eine Schulter angebracht sein, welche als Anschlag für die Mutter dient. An der Mantelfläche 39 der Bohrung 14 im Schaft 1 ist eine parallel zur Längsachse 2 verlaufende Nute 38 angeordnet, in welche ein am Lagerteil 15 radial vorstehender Stift 37 eingreift. Durch die Anordnung der Nute 38 in der Bohrung 14 des Schaftes 1 und des Stiftes 37 am Lagerteil 15 wird erreicht, dass das Lagerteil 15 durch die Spindel 10 axial verschiebbar ist, gegenüber dem Schaft 1 aber gegen Rotation gesichert ist. Zum Drehen der Spindel 10 ist am hinteren Ende 18 derselben ein Innensechskant 19 zur Aufnahme eines entsprechenden Schraubendrehers angebracht. Zudem ist die Spindel 10 am hinteren Ende 18 mit einer koaxialen Bohrung 22 mit Innengewinde 23 versehen, dessen Aussendurchmesser kleiner als der Innensechskantes 19 ist und zur Aufnahme einer Kompressionsschraube (Fig. 3) dient. Am hinteren Schaftende 17 ist ein Schlitz 40 angebracht, welcher zur Aufnahme eines entsprechenden Schraubendrehers dient.

Fig. 3 zeigt die Verwendung des erfindungsgemässen Verankerungselementes in einer Spannvorrichtung zum Fixieren von abgebrochenen Hüftgelenkköpfen 35 am Femur 36. Das Verankerungselement wird mittels der Verankerungsdrähte 7, welche durch Einschrauben der Spindel 15 in die Gewindehülse 11 ausgeknickt werden, in der Spongiosa des Hüftgelenkkopfes 35 verankert. Die am Hauptteil des Femur 36 mittels Knochenschrauben 33 fixierte interne Platte 28 weist eine winklig zur Platte 28 in den Knochen ragende Buchse 29 auf, in deren Bohrung 45 der Schaft 1 des Verankerungselementes parallel zur Längsachse 46 der Bohrung 45 verschiebbar gelagert ist. Mittels einer in das Innengewinde 23 (Fig. 1) an der Spindel 10 einschraubbaren Kompressionsschraube 30, deren Kopf 31 auf einer entsprechenden Schulterfläche 32 am plattenseitigen Ende der Buchse 29 aufliegt, kann das Verankerungselement so fixiert werden, dass vom Hüftgelenkkopf 35 her Zugkräfte auf das Verankerungselement ausgeübt werden können, während ebenfalls vom Hüftgelenkkopf 35 her wirkende Druckkräfte zu einer axialen Verschiebung des Verankerungselementes führen. Die während der Heilung der Fraktur möglicherweise auftretende Verkürzung kann durch diese mögliche Verschiebung des Verankerungselementes in der Buchse 29 aufgefangen werden. Würde das Verankerungselement dieser Verkürzung nicht nachgeben, bestünde die Gefahr, dass der Schaft 1 den Hüftgelenkkopf 35 durchstösst. Ferner ist zwischen dem Verankerungselement 27 und der Buchse 29 eine Verdrehsicherung 50 angebracht, welche eine Rotation des Hüftgelenkkopfes 35 um die Längsachse 2 verhindert. Die Verdrehsicherung 50 umfasst in dieser Ausführungsform der erfindungsgemässen Spannvorrichtung eine Aussenverzahnung 51 am hinteren Schaftabschnitt 4 sowie eine komplementäre Innenverzahung 52 in der Bohrung 45 der Buchse 29.

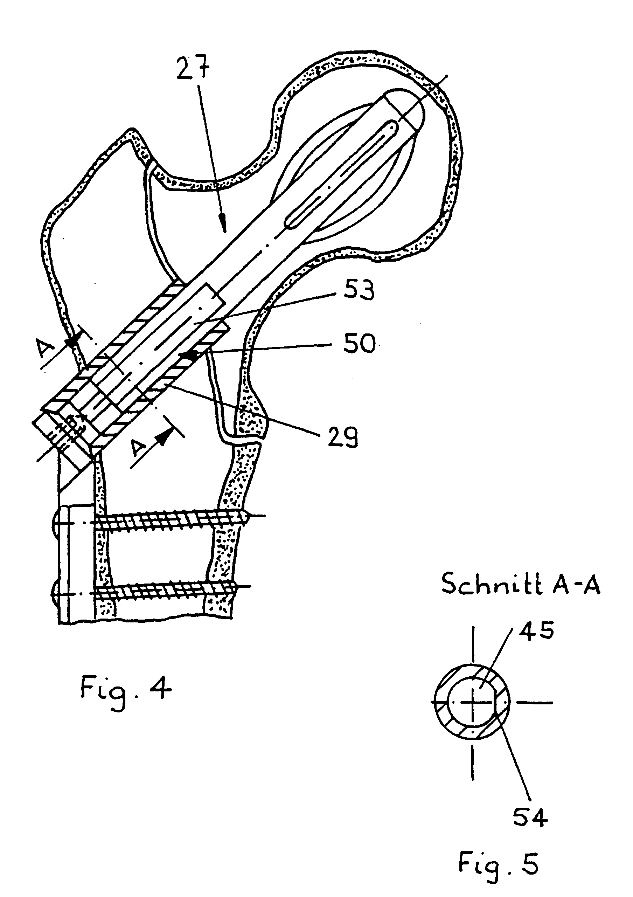

Die Fig. 4 und 5 zeigen eine Ausführungsform der erfindungsgemässen Spannvorrichtung, welche sich von der in Fig. 3 dargestellten Ausführungsform nur darin unterscheidet, dass die Verdrehsicherung 50 zwischen Verankerungselement 27 und Buchse 29 eine ebene Fläche 53, welche auf dem hinteren Schaftabschnitt 4 radial vertieft angebracht ist, und eine dazu komplementäre Erhebung 54 in der Bohrung 45 der Buchse 29 umfasst, so dass der kreisflächenförmige Querschnitt der Bohrung 45 einen kreissegmentförmigen Ausschnitt mit einem Zentriwinkel des Kreissegmentes von 60° enthält.

Anstelle der in den Fig. 3, 4 und 5 dargestellten Ausführungsformen der Verdrehsicherung 50 ist auch eine Ausführung der Verdrehsicherung 50 mit einer Nute und einem komplementären Nocken möglich.

Eine ausführliche Beschreibung der Operationstechnik zur Implantation solcher Spannvorrichtungen ist in den CH 634 741 und CH 634 742 zusammengestellt.

## Patentansprüche

1. Osteosynthetisches Verankerungselement zur Fixation von Knochen, insbesondere eines Hüftgelenkkopfes an einem Femur, mit einem longitudinalen Schaft (1), welcher eine Längsachse (2), einen in einen Knochen einbringbaren vorderen Schaftabschnitt (3) und einen hinteren Schaftabschnitt (4) umfasst, wobei der vordere Schaftabschnitt (3) Verankerungsmittel (5) zur Fixierung des Schaftes (1) in einem Knochen und der hintere Schaftabschnitt (4) eine Verdrehsicherung (50) zur bezüglich der Längsachse (2) rotationsstabilen Aufnahme in einer implantierbaren Knochenplatte (28) oder Verbindungselement (29) umfasst, wobei
A) die Verankerungsmittel (5) quer zur Längsachse (2) des Schaftes (1) ausfahrbar sind; und
B) der hintere Schaftabschnitt (4) Spannmittel (6) umfasst, mittels welcher die Verankerungsmittel (5) ausfahrbar sind und damit das Verankerungselement in einem Knochen fixierbar ist, wobei
C) die Verankerungsmittel (5) parallel zur Längsachse (2) angeordnete Verankerungsdrähte (7) mit einem Durchmesser d, je einem hinteren, spannmittelseitigen Ende (8) und einem vorderen Ende (9) sind, wobei
D) beide Enden (8;9) im Schaft (1) gelagert sind und bei Betätigung des Spannmittels (5) die Verankerungsdrähte (7) zwischen ihren Enden (8;9) senkrecht zur Längsachse (2) bogenförmig ausbiegbar sind,
**dadurch gekennzeichnet, dass**
E) die Enden (8;9) der Verankerungsdrähte (7) kugelartig ausgebildet sind, wobei der Kugeldurchmesser D_{Kugel} ≥ d ist.

2. Verankerungselement nach Anspruch 1, **dadurch gekennzeichnet, dass** der Schaft (2) zylindrisch ist und einen Durchmesser D aufweist.

3. Verankerungselement nach Anspruch 2, **dadurch gekennzeichnet, dass** die Verankerungsmittel (5) bis zu einem Durchmesser Dₘₐₓ > D ausbiegbar sind.

4. Verankerungselement nach einem der Ansprüche 1 bis 3, **dadurch gekennzeichnet, dass** die Verankerungsmittel (5) elastisch ausbiegbar sind.

5. Verankerungselement nach einem der Ansprüche 1 bis 3, **dadurch gekennzeichnet, dass** die Verankerungsmittel (5) plastisch ausbiegbar sind.

6. Verankerungselement nach einem der Ansprüche 3 bis 5, **dadurch gekennzeichnet, dass** das Verhältnis Dₘₐₓ : D zwischen 1,2 und 3,0 beträgt.

7. Verankerungselement nach Anspruch 6, **dadurch gekennzeichnet, dass** das Verhältnis Dₘₐₓ : D zwischen 1,5 und 2,5 beträgt.

8. Verankerungselement nach einem der Ansprüche 1 bis 7, **dadurch gekennzeichnet, dass** die Verankerungsmittel (5) auf einer Länge L ausbiegbar sind und die Länge L zwischen 10 und 60 mm beträgt.

9. Verankerungselement nach Anspruch 8, **dadurch gekennzeichnet, dass** dieses zwischen drei und sechs Verankerungsdrähte (7) umfasst.

10. Verankerungselement nach einem der Ansprüche 1 bis 9, **dadurch gekennzeichnet, dass** der Durchmesser d zwischen 0,5 mm und 2,5 mm beträgt.

11. Verankerungselement nach Anspruch 10, **dadurch gekennzeichnet, dass** der Durchmesser d zwischen 1 mm und 1,5 mm beträgt.

12. Verankerungselement nach einem der Ansprüche 1 bis 11, **dadurch gekennzeichnet, dass** das Spannmittel (6) koaxial zur Längsachse (2) eine Spindel (10) mit einem Aussengewinde (13) und eine Gewindehülse (11) mit einem zum Aussengewinde (13) korrespondierenden Innengewinde (12) umfasst.

13. Verankerungselement nach Anspruch 12, **dadurch gekennzeichnet, dass** der Schaft (1) eine zur Längsachse (2) konzentrische Bohrung (14) umfasst, worin im hinteren Schaftabschnitt (4) koaxial die Gewindehülse (11) und die Spindel (10) angeordnet sind, wobei die Gewindehülse (11) in der Bohrung (14) fest ist.

14. Verankerungselement nach Anspruch 12 oder 13, **dadurch gekennzeichnet, dass**
A) die Spindel (10) gegen den vorderen Schaftabschnitt (3) hin ein in der Bohrung (14) parallel zur Längsachse (2) verschiebbares Lagerteil (15) mit einer Ringnute (16) umfasst, wobei
B) die Spindel (10) axialfest, aber um die Längsachse (2) rotierbar mit dem Lagerteil (15) verbunden ist;
C) die Mantelfläche (39) der Bohrung (14) im Schaft (1) eine parallel zur Längsachse (2) verlaufende Nute (38) umfasst;
D) das Lagerteil (15) einen radial vorstehenden Stift (37) umfasst, weicher in die Nute (38) eingreift, und
E) die Ringnute (16) in einem Querschnitt senkrecht zur Längsachse (2) angeordnet ist und zur Aufnahme der hinteren Enden (8) der Verankerungsdrähte (7) dient.

15. Verankerungselement nach Anspruch 14, **dadurch gekennzeichnet, dass** die Ringnute (16) einen kreisförmigen Querschnitt aufweist.

16. Verankerungselement nach einem der Ansprüche 12 bis 15, **dadurch gekennzeichnet, dass** der hintere Schaftabschnitt (4) ein hinteres, longitudinal vom vorderen Schaftabschnitt (3) abgewandtes Schaftende (17) aufweist und die Spindel (10) ein hinteres Spindelende (18) aufweist, welches mit dem hinteren Schaftende (17) korrespondiert, und am hinteren Spindelende (18) Mittel (21) zur Aufnahme eines Schraubendrehers umfasst.

17. Verankerungselement nach einem der Ansprüche 12 bis 16, **dadurch gekennzeichnet, dass** der hintere Schaftabschnitt (4) ein hinteres, longitudinal vom vorderen Schaftabschnitt (3) abgewandtes Schaftende (17) aufweist und die Spindel (10) ein hinteres Spindelende (18) aufweist, welches mit dem hinteren Schaftende (17) korrespondiert, und vom hinteren Spindelende (18) her eine konzentrisch zur Längsachse (2) angebrachte Bohrung (22) mit einem Innengewinde (23) zur Aufnahme einer Kompressionsschraube (30).

18. Verankerungselement nach einem der Ansprüche 1 bis 17, **dadurch gekennzeichnet, dass** der vordere Schaftabschnitt (3) ein vorderes, longitudinal vom hinteren Schaftabschnitt (4) abgewandtes Schaftende (20) aufweist und das Verankerungselement an diesem vorderen Schaftende (20) einen Zapfen (24) umfasst, welcher in die Bohrung (14) eingefügt ist und gegen das vordere Schaftende (20) sphärisch ausgebildet ist.

19. Verankerungselement nach Anspruch 18, **dadurch gekennzeichnet, dass** der Zapfen (24) auf seinem in die Bohrung (14) eingefügten Abschnitt (25) eine Ringnute (26) umfasst, wobei die Ringnute (26) in einem Querschnitt senkrecht zur Längsache (2) angeordnet ist und zur Aufnahme der vorderen Enden (9) der Verankerungsdrähte (7) dient.

20. Verankerungselement nach Anspruch 19, **dadurch gekennzeichnet, dass** die Ringnute (26) einen kreisförmigen Querschnitt aufweist.

21. Verankerungselement nach Anspruch 1 oder 2, **dadurch gekennzeichnet, dass** der Schaft (1) prismatisch ist.

22. Verankerungselement nach einem der Ansprüche 1 bis 21, **dadurch gekennzeichnet, dass** der Schaft (1) an seinem hinteren Schaftende (17) Mittel zur Aufnahme eines Schraubendrehers aufweist.

23. Spannvorrichtung mit einem Verankerungselement nach einem der Ansprüche 1 bis 22 zum Fixieren abgebrochener Hüftgelenkköpfe, **dadurch gekennzeichnet, dass** die Spannvorrichtung folgende Elemente umfasst:
A) das in dem abgebrochenen Gelenkkopf (35) zu befestigende Verankerungselement (27);
B) eine am Hauptteil des Femur (36) festzuschraubende Platte (28) mit einer winklig zur Platte (28) angeordneten Buchse (29), worin das Verankerungselement (27) mit seinem hinteren Schaftabschnitt (4) aufnehmbar und koaxial zur Längsachse (2) verschiebbar ist; sowie
C) eine in das Innengewinde (23) der Spindel (10) einschraubbare Kompressionsschraube (30), deren Kopf (31) auf der Schulterfläche (32) der Buchse (29) aufliegt; und
D) eine Verdrehsicherung (50) zwischen dem Verankerungselement (27) und der Buchse (29).

24. Spannvorrichtung nach Anspruch 23, **dadurch gekennzeichnet, dass** sie zusätzlich Knochenschrauben (33) zur Fixation der Platte (28) am Femur (36) umfasst.

## Claims

1. An osteosynthetic anchoring member for fixating bones, in particular a femoral head to a femur, including a longitudinal shaft (1), which comprises a longitudinal axis (2), a front shaft portion (3) insertable into the bone and a rear shaft portion (4), the front shaft portion (3) comprising anchoring means (5) for fixating the shaft (1) in a bone and the rear shaft portion (4) comprising anti-rotation means (50) for enabling said rear shaft portion to be received in an implantable bone plate (28) or a connecting member (29) in such a way as to be secured against rotating about the longitudinal axis (2), whereby
A) the anchoring means (5) are capable of being extended vertically to the longitudinal axis (2) of the shaft (1), and
B) the rear shaft portion (4) comprises tension means (6) by means of which the anchoring means (5) may be extended, thus enabling the anchoring member to be fixated within a bone, whereby
C) the anchoring means (5) consist of anchoring wires (7) arranged parallel to the longitudinal axis (2) having a diameter d, a rear end (8) facing the tension means and a front end (9), whereby
D) both ends (8;9) being mounted within the shaft (1) and the anchoring wires (7) being apt to bend outward in an arch-shaped manner vertically to the longitudinal axis (2) as the tension means (5) is actuated,
**characterised in that**
E) the ends (8;9) of the anchoring wires (7) are shaped in the form of balls, with the ball diameter D_{ball} being ≥ d.

2. An anchoring member as claimed in claim 1, **characterised in that** the shaft (1) is cylindrical and has a diameter D.

3. An anchoring member as claimed in claim 2, **characterised in that** the anchoring means (5) may be bent outward so as to achieve a diameter Dₘₐₓ > D.

4. An anchoring member as claimed in any of the claims 1 to 3, **characterised in that** the anchoring means (5) are apt to resiliently be bent outward.

5. An anchoring member as claimed in any of the claims 1 to 3, **characterised in that** the anchoring means (5) apt to be bent outward are subject to plastic deformation.

6. An anchoring member as claimed in any of the claims 3 to 5, **characterised in that** the ratio Dₘₐₓ : D is between 1.2 and 3.0.

7. An anchoring member as claimed in claim 6, **characterised in that** the ratio Dₘₐₓ : D is between 1.5 and 2.5.

8. An anchoring member as claimed in any of the claims 1 to 7, **characterised in that** the anchoring means (5) may be bent outward over a length L and that the length L is between 10 mm and 60 mm.

9. An anchoring member as claimed in claim 8, **characterised in that** said anchoring member comprises between three and six anchoring wires (7).

10. An anchoring member as claimed in any of the claims 1 to 9, **characterised in that** the diameter d is between 0.5 mm and 2.5 mm.

11. An anchoring member as claimed in claim 10, **characterised in that** the diameter d is between 1 mm and 1.5 mm.

12. An anchoring member as claimed in any of the claims 1 to 11, **characterised in that** the tension means (6) comprises, in coaxial alignment with the longitudinal axis (2), a spindle (10) with an external screw thread (13) and a threaded sleeve (11) with an internal screw thread (12) corresponding to the external screw thread (13).

13. An anchoring member as claimed in claim 12, **characterised in that** the shaft (1) comprises a bore (14) extending concentrically to the longitudinal axis (2) in which bore the threaded sleeve (11) and the spindle (10) are arranged coaxially in the rear shaft portion (4), the threaded sleeve (11) being in fixed engagement with the bore (14).

14. An anchoring member as claimed in claim 12 or 13, **characterised in that**
A) the spindle (10) comprises a bearing member (15) with an annular groove (16) located towards the front shaft portion (3) and displaceable within the bore (14) parallel to the longitudinal axis (2),
B) the spindle (10) being connected to the bearing member (15) in such a way as to be secured against axial displacement while being capable of rotating about the longitudinal axis (2);
C) the lateral area (39) of the bore (14) formed in the shaft (1) comprises a groove (38) extending parallel to the longitudinal axis (2);
D) the bearing member (15) comprises a radially projecting finger (37) which engages with said groove (38), and
E) the annular groove (16) is arranged in a cross-section extending vertically to the longitudinal axis (2) and serves for receiving the rear ends (8) of the anchoring wires (7).

15. An anchoring member as claimed in claim 14, **characterised in that** the annular groove (16) has a circular cross-section.

16. An anchoring member as claimed in any of the claims 12 to 15, **characterised in that** the rear shaft portion (4) has a rear shaft end (17) longitudinally opposite to the front shaft portion (3), and that the spindle (10) has a rear spindle end (18) which corresponds to the rear shaft end (17) and comprises means (21) for receiving a screw driver.

17. An anchoring member as claimed in any of the claims 12 to 16, **characterised in that** the rear shaft portion (4) has a rear shaft end (17) longitudinally opposite to the front shaft portion (3), and that the spindle (10) has a rear spindle end (18) which corresponds to the rear shaft end (17) and comprises a bore (22) extending from the rear spindle end (18) concentrically to the longitudinal axis (2) and including an internal screw thread (23) for receiving a compression bone screw (30).

18. An anchoring member as claimed in any of the claims 1 to 17, **characterised in that** the front shaft portion (3) has a front shaft end (20) longitudinally opposite to the rear shaft portion (4) and that in the front shaft end (20) the anchoring member comprises a plug member (24) which is inserted into the bore (14) and which, in its portion facing the front shaft end (20), is spherically shaped.

19. An anchoring member as claimed in claim 18, **characterised in that** the plug member (24) in its portion (25) inserted into the bore (14) comprises an annular groove (26), the annular groove (26) being arranged in a cross-section vertical to the longitudinal axis (2) and serving for receiving the front ends (9) of the anchoring wires (7).

20. An anchoring member as claimed in claim 19, **characterised in that** the annular groove (26) has a circular cross-section.

21. An anchoring member as claimed in claim 1 or 2, **characterised in that** the shaft (1) has a prismatic form.

22. An anchoring member as claimed in any of the claims 1 to 21, **characterised in that** the shaft (1) on its rear shaft end (17) includes means for receiving a screw driver.

23. A fixation device for fixating fractured femoral heads including an anchoring member according to any of the claims 1 to 22, **characterised in that** the fixation device comprises the following elements:
A) the anchoring member (27) to be fixed within the fractured femoral head (35);
B) a plate (28) to be screwed to the main part of the femur (36) including a sleeve (29) extending at an angle with the plate (28) in which the rear shaft portion (4) of the anchoring member (27) may be received and wherein it is displaceable coaxially to the longitudinal axis (2);
C) a compression bone screw (30) to be screwed into the internal screw thread (23) of the spindle (10), the head (31) of which is supported by the shoulder surface (32) of the sleeve (29); and
D) an anti-rotation means (50) located between the anchoring member (27) and the sleeve (29).

24. A fixation device as claimed in claim 23, **characterised in that** in addition said fixation device comprises bone screws (33) for fixating the plate (28) to the femur (36).

## Revendications

1. Elément d'ancrage pour ostéosynthèse permettant de fixer des os, notamment une tête fémorale sur un fémur, comportant un corps longitudinal (1) qui comprend un axe longitudinal (2), une partie antérieure (3) pouvant être introduite dans un os et une partie postérieure (4), la partie antérieure (3) dudit corps comprenant des moyens de fixation (5) permettant de fixer le corps (1) dans un os et la partie postérieure (4) dudit corps comprenant un dispositif anti-torsion (50) assurant une réception dans une plaque d'ostéosynthèse implantable (28) ou dans un élément d'assemblage (29) de manière à éviter toute rotation par rapport à l'axe longitudinal (2),
A) les moyens d'ancrage (5) pouvant être sortis transversalement à l'axe longitudinal (2) du corps (1); et
B) la partie postérieure (4) comprenant des moyens de tension (6) grâce auxquels les moyens d'ancrage (5) peuvent être sortis, assurant ainsi la fixation de l'élément d'ancrage dans un os,
C) les moyens d'ancrage (5) étant des fils d'ancrage (7) d'un diamètre d disposés parallèlement à l'axe longitudinal (2) et comprenant respectivement une extrémité postérieure (8) située du côté des moyens de tension et une extrémité antérieure (9),
D) les deux extrémités (8;9) étant logées à l'intérieur du corps (1) et les fils d'ancrage (7) pouvant être, lors de l'actionnement du moyen de tension (6), recourbés vers l'extérieur de manière à former un arc entre leurs extrémités (8;9), et ce perpendiculairement à l'axe longitudinal (2), **caractérisé en ce que**
E) les extrémités (8;9) des fils d'ancrage (7) sont réalisées sous forme sphérique, le diamètre des sphères D_{sphère} étant ≥ d.

2. Elément d'ancrage selon la revendication 1, **caractérisé en ce que** le corps (1) est de forme cylindrique et présente un diamètre D.

3. Elément d'ancrage selon la revendication 2, **caractérisé en ce que** les moyens d'ancrage (5) peuvent être recourbés vers l'extérieur jusqu'à un diamètre Dₘₐₓ > D.

4. Elément d'ancrage selon l'une des revendications 1 à 3, **caractérisé en ce que** les moyens d'ancrage (5) peuvent être recourbés vers l'extérieur de manière élastique.

5. Elément d'ancrage selon l'une des revendications 1 à 3, **caractérisé en ce que** les moyens d'ancrage (5) peuvent être recourbés vers l'extérieur de manière plastique.

6. Elément d'ancrage selon l'une des revendications 3 à 5, **caractérisé en ce que** le rapport Dₘₐₓ: D est compris entre 1,2 et 3,0.

7. Elément d'ancrage selon la revendication 6, **caractérisé en ce que** le rapport Dₘₐₓ: D est compris entre 1,5 et 2,5.

8. Elément d'ancrage selon l'une des revendications 1 à 7, **caractérisé en ce que** les moyens d'ancrage (5) peuvent être recourbés vers l'extérieur sur une longueur L et **en ce que** la longueur L est comprise entre 10 et 60 mm.

9. Elément d'ancrage selon la revendication 8, **caractérisé en ce que** celui-ci comprend entre trois et six fils d'ancrage (7).

10. Elément d'ancrage selon l'une des revendications 1 à 9, **caractérisé en ce que** le diamètre d est compris entre 0,5 mm und 2,5 mm.

11. Elément d'ancrage selon la revendication 10, **caractérisé en ce que** le diamètre d est compris entre 1 mm et 1,5 mm.

12. Elément d'ancrage selon l'une des revendications 1 à 11, **caractérisé en ce que** le moyen de tension (6) comprend, disposée de manière coaxiale à l'axe longitudinal (2), une tige (10) pourvue d'un filet extérieur (13) et un manchon taraudé (11) pourvu d'un filet intérieur (12) correspondant au filet extérieur (13).

13. Elément d'ancrage selon la revendication 12, **caractérisé en ce que** le corps (1) comprend un trou (14) concentrique à l'axe longitudinal (2), dans lequel sont disposés de manière coaxiale, dans la partie postérieure (4) dudit corps, le manchon taraudé (11) et la tige (10), le manchon taraudé (11) étant disposé de manière fixe dans le trou (14).

14. Elément d'ancrage selon la revendication 12 ou 13, **caractérisé en ce que**
A) la tige (10) comprend, vers la partie antérieure (3) du corps dudit élément d'ancrage, un élément d'appui (15) coulissant dans le trou (14) parallèlement à l'axe longitudinal (2) et comportant une rainure annulaire (16),
B) la tige (10) étant reliée à l'élément d'appui (15) de manière fixe quant au déplacement axial, tout en permettant, cependant, un mouvement de rotation sur l'axe longitudinal (2);
C) la surface latérale (39) du trou (14) ménagé dans le corps (1) comprend une rainure (38) s'étendant parallèlement à l'axe longitudinal (2);
D) l'élément d'appui (15) comprend une cheville (37) saillant de manière radiale, qui s'engage dans la rainure (38), et
E) la rainure annulaire (16) est disposée dans un plan perpendiculaire à l'axe longitudinal (2) et sert à recevoir les extrémités postérieures (8) des fils d'ancrage (7).

15. Elément d'ancrage selon la revendication 14, **caractérisé en ce que** la rainure annulaire (16) présente une section circulaire.

16. Elément d'ancrage selon l'une des revendications 12 à 15, **caractérisé en ce que** la partie postérieure (4) du corps dudit élément d'ancrage présente une extrémité postérieure (17) opposée longitudinalement à la partie antérieure (3) dudit corps, et **en ce que** la tige (10) présente une extrémité postérieure (18) qui correspond à l'extrémité postérieure (17) dudit corps et comprend, à son extrémité postérieure (18), des moyens (21) permettant de recevoir un tournevis.

17. Elément d'ancrage selon l'une des revendications 12 à 16, **caractérisé en ce que** la partie postérieure (4) du corps dudit élément d'ancrage présente une extrémité postérieure (17) opposée longitudinalement à la partie antérieure (3), et **en ce que** la tige (10) présente une extrémité postérieure (18) correspondant à l'extrémité postérieure (17) dudit corps, ainsi qu'un trou (22) ménagé dans ladite tige à partir de son extrémité postérieure (18) de manière concentrique à l'axe longitudinal (2) et pourvu d'un filet intérieur (23) destiné à recevoir une vis de compression (30).

18. Elément d'ancrage selon l'une des revendications 1 à 17, **caractérisé en ce que** la partie antérieure (3) du corps dudit élément d'ancrage présente une extrémité antérieure (20) opposée longitudinalement à la partie postérieure (4), et **en ce que** l'élément d'ancrage comprend, sur cette extrémité antérieure (20), un tenon (24) qui est inséré dans le trou (14) et dont la partie orientée vers l'extrémité antérieure (20) dudit corps présente une forme sphérique.

19. Elément d'ancrage selon la revendication 18, **caractérisé en ce que** le tenon (24) comprend, sur sa partie (25) qui est insérée dans la trou (14), une rainure annulaire (26), la rainure annulaire (26) étant disposée dans un plan perpendiculaire à l'axe longitudinal (2) et servant à recevoir les extrémités antérieures (9) des fils d'ancrage (7).

20. Elément d'ancrage selon la revendication 19, **caractérisé en ce que** la rainure annulaire (26) présente une section circulaire.

21. Elément d'ancrage selon la revendication 1 ou 2, **caractérisé en ce que** le corps (1) est de forme prismatique.

22. Elément d'ancrage selon l'une des revendications 1 à 21, **caractérisé en ce que** le corps (1) présente, sur son extrémité postérieure (17), des moyens destinés à recevoir un tournevis.

23. Dispositif de tension comportant un élément d'ancrage selon l'une des revendications 1 à 22 permettant de fixer des têtes fémorales fracturées, **caractérisé en ce que** le dispositif de tension comprend les éléments suivants :
A) l'élément d'ancrage (27) devant être fixé dans la tête fémorale fracturée (35);
B) une plaque (28) destinée à être vissée sur la partie principale du fémur (36) et comportant un manchon (29) formant un angle avec la plaque (28) et dans lequel peut être logée la partie postérieure (4) du corps de l'élément d'ancrage (27) et dans lequel ce dernier peut coulisser de manière coaxiale à l'axe longitudinal (2); ainsi que
C) une vis de compression (30) pouvant être vissée dans le filet intérieur (23) de la tige (10) et dont la tête (31) repose sur la surface d'épaulement (32) du manchon (29); et
D) un dispositif anti-torsion (50) entre l'élément d'ancrage (27) et le manchon (29).

24. Dispositif de tension selon la revendication 23, **caractérisé en ce que** celui-ci comprend, en outre, des vis d'ostéosynthèse (33) permettant de fixer la plaque (28) sur le fémur (36).
